(19) Europäisches Patentamt
European Patent Office
Office européen des brevets

(11) Numéro de publication : **0 480 794 A1**

(12) **DEMANDE DE BREVET EUROPEEN**

(21) Numéro de dépôt : **91402604.2**

(51) Int. Cl.$^5$ : **C07D 401/12,** A61K 31/505

(22) Date de dépôt : **30.09.91**

(30) Priorité : **02.10.90 FR 9012094**

(43) Date de publication de la demande : **15.04.92 Bulletin 92/16**

(84) Etats contractants désignés : **AT BE CH DE DK ES FR GB GR IT LI LU NL SE**

(71) Demandeur : **SYNTHELABO**
**58 rue de la Glacière**
**F-75013 Paris (FR)**

(72) Inventeur : **George, Pascal**
**19, rue des Quatre Vents**
**F-78730 St Arnoult en Yvelines (FR)**
Inventeur : **Maloizel, Christian**
**21, rue du Plateau**
**F-92190 Meudon (FR)**
Inventeur : **Marabout, Benoit**
**6, rue Georges Ritz**
**F-91300 Massy (FR)**

(74) Mandataire : **Thouret-Lemaitre, Elisabeth et al**
**SYNTHELABO Service Brevets 22, avenue Galilée, B.P. Box 72**
**F-92352 LE PLESSIS ROBINSON CEDEX (FR)**

(54) **Dérivés de 2-aminopyrimidine-4-carboxamide, leur préparation et leur application en thérapeutique.**

(57) Dérivés de 2-aminopyrimidine-4-carboxamide, sous la forme de racémates ou d'énantiomères, répondant à la formule (I)

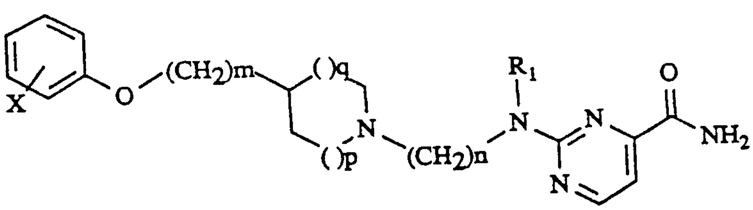

(I)

dans laquelle
m représente le nombre 0 ou 1,
p représente le nombre 0 ou 1,
q représente le nombre 1 ou 2,
n représente le nombre 2 ou 3,
$R_1$ représente un atome d'hydrogène ou un groupe méthyle, et
X représente un ou plusieurs atomes ou groupes choisis parmi les suivants : hydrogène, fluor, chlore, méthoxy, méthyle, isopropyle,
ainsi que leurs sels d'addition aux acides pharmaceutiquement acceptables.
Application en thérapeutique.

EP 0 480 794 A1

La présente invention a pour objet des dérivés de 2-aminopyrimidine-4-carboxamide, leur préparation et leur application en thérapeutique.

Les composés de l'invention répondent à la formule générale I du schéma donné en annexe dans laquelle
m représente le nombre 0 ou 1,
p représente le nombre 0 ou 1,
q représente le nombre 1 ou 2,
n représente le nombre 2 ou 3,
$R_1$ représente un atome d'hydrogène ou un groupe méthyle, et
X représente un ou plusieurs atomes ou groupes choisis parmi les suivants : hydrogène, fluor, chlore, méthoxy, méthyle, isopropyle.

Les composés de l'invention peuvent se présenter à l'état de bases libres ou de sels d'addition à des acides pharmaceutiquement acceptables, sous forme de mélanges racémiques ou d'énantiomères purs.

Conformément à l'invention, on peut préparer les composés de formule générale I selon le procédé illustré par le schéma 1 donné en annexe.
On fait réagir une amine de formule (II) (dans laquelle X, m, p et q sont tels que définis ci-dessus), éventuellement sous forme de chlorhydrate, avec un réactif halogéné de formule (III) (dans laquelle y représente un atome d'halogène, n est tel que défini ci-dessus et, soit $R_1$ est tel que défini ci-dessus et R représente un groupe protecteur de l'amine par exemple un groupe triphénylméthyle, soit $R_1$ et R forment ensemble un groupe protecteur tel que le groupe phtalimido comme décrit dans J. Med. Chem (1989), 32(8), 1921-1926 et Chem. Pharm. Bull (1989), 37(1), 100-105. La réaction s'effectue dans un solvant aprotique tel que le diméthylformamide (DMF) en présence d'une base organique telle que la triéthylamine (TEA) ou minérale telle que le carbonate de potassium ($K_2CO_3$) à une température de 40 à 100°C.
On obtient une diamine de formule (IV) et on déprotège l'alkylamine terminale : dans le cas où R est un groupe triphénylméthyle, on effectue un traitement à l'acide chlorhydrique gazeux dans un alcool aliphatique, par exemple le méthanol, à une température de 0 à 60°C ; dans le cas où R et $R_1$ forment ensemble un groupe phtalimido, on effectue un traitement analogue à celui décrit dans la littérature citée ci-dessus, par exemple avec l'hydrazine.
On obtient ainsi l'amine de formule (V) que l'on fait réagir avec un 2-chloropyrimidine-4-carboxamide de formule (VI) dans un solvant aprotique, par exemple le DMF, en présence d'une base, par exemple $K_2CO_3$, à une température de 20 à 40°C pour arriver au dérivé de 2-aminopyrimidine-4-carboxamide de formule (I).

Les phénoxypipéridines (II) peuvent être obtenues par des méthodes analogues à celles décrites dans le brevet US 4031221, J. Med. Chem. (1974) 17(9) 1000-1008 et le brevet DE 1964515. Les phénoxyméthylpipéridines (II) peuvent être préparées par des méthodes analogues à celles décrites dans J. Med. Chem. (1987) 30(1) 222-5 et des brevets DE 2737630 DE 2549999, GB 1203149 et FR 2010615.

Les ω-halogéno-N-(triphénylméthyl)alkylamines (III) et le 2-chloropyrimidine-4-carboxamide sont décrits dans le brevet FR 8917304.

Le réactif halogéné de formule (III) est soit disponible dans le commerce quand $R_1$ et R forment ensemble un groupe phtalimido, soit, quand $R_1$ est H ou $CH_3$, peut être préparé suivant le schéma 2, donné ci-après selon lequel on fait réagir une-ω-halogénoalkylamine de formule (VII) avec un composé de formule RCl, en l'occurrence le chlorure de trityle, dans un solvant inerte halogéné tel que le dichlorométhane, en présence d'une base organique telle que la triéthylamine, à une température de 20 à 80°C.

schéma 2

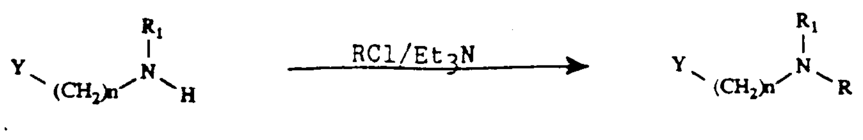


(VII) (III)

Le 2-chloropyrimidine-4-carboxamide de formule (VI) peut être préparé selon le schéma 3, donné ci-après, à partir du 2-chloropyrimidine-4-carbonitrile (VIII) par traitement à l'acide chlorhydrique gazeux dans l'acide formique. Le 2-chloropyrimidine-4-carbonitrile est préparé selon la méthode décrite dans J. Het. Chem. (1964),

1, 130-133.

schéma 3

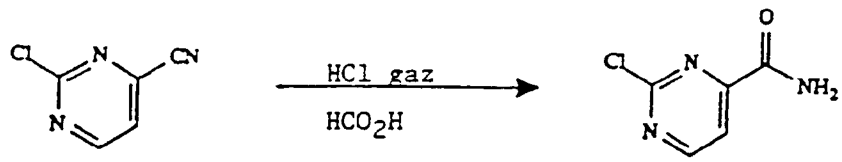


(VIII) (VI)

Les exemples suivants illustrent la préparation de quelques composés selon l'invention.
Les microanalyses élémentaires et les spectres IR et RMN confirment les structures des produits obtenus.

Exemple 1. 2-[[3-[4-(4-fluorophénoxy)pipéridin-1-yl]propyl] amino]pyrimidine-4-carboxamide.

1.1. 2-[3-[4-(4-fluorophénoxy)pipéridin-1-yl]propyl]-1H-isoindole-1,3(2H)-dione.
On fait réagir 9,26 g (0,04 M) de chlorhydrate de 4-(4-fluorophénoxy)pipéridine, 10,72 g (0,04 M) de 3-bromopropyl-N-phtalimide et 13,89 g (0,1 M) de $K_2CO_3$ dans 90 ml de DMF pendant 3 h à 100°C.
On verse le mélange réactionnel dans de l'eau glacée. On l'extrait 3 fois avec AcOEt puis lave la phase organique 3 fois à l'eau puis 1 fois à l'eau saturée de NaCl.
On sèche la solution sur $MgSO_4$, filtre et concentre.
On obtient le composé. Rendement : 100 %.
1.2. 4-(4-fluorophénoxy)pipéridine-1-propanamine.
On fait réagir 13,8 g (0,04 M) de 2-[3-[4-(4-fluorophénoxy)pipéridin-1-yl]propyl]-1H-isoindole-1,3(2H)-dione et 3,9 ml (0,08 M) d'hydrazine dans 280 ml de EtOH pendant 3 h à la température du reflux puis on abandonne le mélange 1 nuit. On introduit alors 15 ml de HCl concentré et 20 ml de $H_2O$. On laisse le mélange 3 h à la température du reflux puis on filtre et concentre. On reprend le résidu à l'eau, filtre et rince à l'eau. On lave le filtrat 2 fois à l'éther puis on l'alcalinise avec NaOH et on extrait 3 fois avec $CH_2Cl_2$. On sèche la solution sur $Na_2SO_4$, filtre et concentre.
On obtient le composé. Rendement : 86 %.
1.3. 2-[[3-[4-(4-fluorophénoxy)pipéridin-1-yl]propyl]amino]pyrimidine-4-carboxamide.
On fait réagir 3,8 g (0,015 M) de 4-(4-fluorophénoxy)pipéridine-1-propanamine, 2,36 g (0,015 M) de 2-chloropyrimidine-4-carboxamide et 2,07 g (0,0225 M) de $K_2CO_3$ dans 80 ml de DMF. On agite le mélange pendant 48 h à la température ambiante. On le verse dans de l'eau glacée et on extrait 3 fois avec de l'acétate d'éthyle (AcOEt). On lave la phase organique 3 fois à l'eau puis sèche sur $MgSO_4$, filtre et concentre.
On obtient 5 g (0,0133 M) de base que l'on reprend dans un mélange EtOH + $CH_2Cl_2$ et on ajoute une solution de 1,55 g (0,0133 M) d'acide fumarique dans 200 ml d'EtOH. On concentre au 1/3 puis laisse cristalliser. Rendement 61 %.
F = 171-174°C.

Exemple 2. 2-[[3-[3-(phénoxyméthyl)pipéridin-1-yl]propyl]amino]pyrimidine-4-carboxamide.

2.1. 2-[3-[3-(phénoxyméthyl)pipéridin-1-yl]propyl]-1H-isoindole-1,3(2H)-dione.
Dans un ballon tricol de 150 ml, on introduit 6,8 g (0,03 M) de chlorhydrate de 3-(phénoxyméthyl)pipéridine, 8 g (0,03 M) de 3-bromopropyl-N-phtalimide et 10,4 g (0,075 M) de $K_2CO_3$ en solution dans 85 ml de DMF.
On agite le mélange à 100°C pendant 2 h.
On le verse dans de l'eau glacée, l'extrait 3 fois avec AcOEt puis lave 3 fois à l'eau la phase organique et une dernière fois avec de l'eau saturée de NaCl.
On sèche la solution sur $MgSO_4$, filtre et concentre.
On obtient 12 g d'huile (rendement 100 %).
2.2. 3-(phénoxyméthyl)pipéridine-1-propanamine.
A 11,3 g (0,03 M) de 2-[3-[3-(phénoxyméthyl)pipéridin-1-yl]propyl]-1H-isoindole-1,3(2H)-dione dans 226 ml d'EtOH, on ajoute 2,9 ml (0,06 M) d'hydrazine. On chauffe le mélange à la température du reflux tout en agitant

pendant 3 h. On le laisse refroidir et filtre, on rince avec EtOH puis concentre le filtrat. On reprend le résidu à l'éther. On concentre le filtrat et obtient 4,6 g d'huile jaune. Les 2 insolubles sont repris dans 100 ml de $H_2O$ et 25 ml de HCl concentré. On chauffe le mélange au reflux tout en agitant pendant 2 h 30. On laisse refroidir la solution. On filtre, rince avec un peu d'eau puis alcalinise toute la phase par NaOH (lessive de soude). On extrait à l'éther 3 fois et lave 1 fois à l'eau. On sèche la solution sur $Na_2SO_4$ puis filtre et concentre. On obtient 2,3 g d'huile.

2.3. 2-[[3-[3-(phénoxyméthyl)pipéridin-1-yl]propyl]amino]pyrimidine-4-carboxamide.

Dans un ballon de 250 ml on introduit 3,45 g (0,0147 M) de 3-(phénoxyméthyl)pipéridine-1-propanamine, 2,32 g (0,0147 M) de 2-chloropyrimidine-4-carboxamide et 3 g (0,022 M) de $K_2CO_3$ dans 35 ml de DMF. On agite le mélange à la température ambiante pendant 48 h. On le verse dans de l'eau et l'extrait 3 fois avec AcOEt. La phase organique est lavée 4 fois à l'eau, on sèche la solution sur $MgSO_4$, filtre et concentre. On reprend l'huile obtenue à l'éther et on filtre.

On obtient 5,3 g (0,0143 M) de base que l'on reprend dans un peu d'EtOH. On y ajoute une solution de 1,66 g (0,0143 M) d'acide fumarique en solution dans 200 ml d'EtOH. On concentre presqu'à sec puis ajoute un peu d'éther. Le produit cristallise. On le filtre et le fait recristalliser dans de l'EtOH. Rendement : 66 %. F = 144-146°C.

<u>Exemple 3.</u> 2-[[3-[3-[(4-fluorophénoxy)méthyl]pipéridin-1-yl]propyl]amino]pyrimidine-4-carboxamide.

3.1. 3-[(4-fluorophénoxy)méthyl]-<u>N</u>-(triphénylméthyl)pipéridine-1-propanamine.

On fait réagir 6,14 g (0,025 M) de chlorhydrate de 3-[(4-fluorophénoxy)méthyl]pipéridine, 10,46 g (0,0275 M) de 3-bromo-<u>N</u>-trihénylméthylpropanamine et 8,64 g (0,0625 M) de carbonate de potassium dans 50 ml de DMF. On chauffe le mélange réactionnel à 90-95°C pendant 7 h 30. On refroidit le mélange réactionnel à la température ambiante, le verse sur 150 ml d'eau et l'extrait avec 3 fois 100 ml d'AcOEt. On réunit les phases organiques, les lave à l'eau et les sèche. On filtre et évapore. On obtient le produit après chromatographie.

3.2. Chlorhydrate de 3-[(4-fluorophénoxy)méthyl]pipéridine-1-propanamine.

On met en suspension dans 300 ml de méthanol, 9,9 g (0,0195 M) de 3-[(4-fluorophénoxy)méthyl]-<u>N</u>-(triphénylméthyl)pipéridine-1-propanamine et on fait buller de l'acide chlorhydrique gazeux pendant 20 mn en refroidissant le mélange réactionnel avec un mélange glace/sel/eau. On chauffe le mélange réactionnel à la température du reflux pendant 5 h puis on le refroidit à la température ambiante. On évapore et filtre. Après recristallisation et séchage on obtient 5,81 g de produit.

F = 130-134°C.

3.3. 2-[[3-[3-[(4-fluorophénoxy)méthyl]pipéridin-1-yl]propyl]amino]pyrimidine-4-carboxamide.

On met en suspension dans 450 ml de DMF, 5,09 g (0,015 M) de chlorhydrate de 3-[(4-fluorophénoxy)méthyl]pipéridine-1-propanamine, 2,36 g de 2-chloropyrimidine-4-carboxamide, 7,26 g (0,0525 M) de $K_2CO_3$ et 0,2 g d'iodure de sodium. On agite le mélange réactionnel sous argon pendant 21 h, le verse sur 200 ml d'eau et l'extrait 3 fois avec de l'acétate d'éthyle. On lave la phase organique à l'eau, sèche, filtre et évapore. On obtient 3,35 g de produit que l'on fait réagir avec 1 g d'acide fumarique dans un mélange éthanol/oxyde d'éthyle. On fait recristalliser le fumarate dans de l'éthanol. Rendement : 51,65 % - F = 150-153°C.

<u>Exemple 4.</u> 2-[[3-[4-[[5-méthyl-2-(1-méthyléthyl)phénoxy]méthyl]pipéridin-1-yl]propyl]amino]pyrimidine-4-carboxamide.

4.1. 4-[[5-méthyl-2-(1-méthyléthyl)phénoxy]méthyl]-<u>N</u>-(triphénylméthyl)pipéridine-1-propanamine.

On fait réagir 11,35 g (0,04 M) de chlorhydrate de 4-[[5-méthyl-2-(1-méthyléthyl)phénoxy]méthyl]pipéridine, 10,72 g (0,04 M) de 3-bromopropyl-N-phtalamide et 13,8 g (0,1 M) de $K_2CO_3$ dans 113 ml de DMF. On agite le mélange pendant 3 h à 100°C. On le verse dans de l'eau glacée. On extrait la solution avec AcOEt puis lave à l'eau. On sèche, filtre et concentre.

4.2. Chlorhydrate de 4-[[5-méthyl-2-(1-méthyléthyl)phénoxy]méthyl]pipéridine-1-propanamine.

On fait réagir 17,35 g (0,04 M) de 4-[[5-méthy1-2-(1-méthyléthyl)phénoxy]méthyl]-<u>N</u>-(triphénylméthyl)pipéridine-1-propanamine dans 340 ml d'EtOH avec 3,9 ml (0,08 M) d'hydrazine. On porte le mélange à la température du reflux pendant 3 h.

On filtre, rince avec un peu d'EtOH, on concentre le filtrat puis le reprend dans de l'éther. On filtre à nouveau un insoluble. On concentre le filtrat. Les insolubles sont réunis dans un ballon et on ajoute 25 ml d'HCl concentré et 75 ml d'$H_2O$. Tout en agitant on porte au reflux pendant 2 h. On laisse refroidir puis filtre, rince avec de l'eau puis on alcalinise par $NH_4OH$ concentrée et extrait 3 fois à l'éther. On sèche la solution sur $Na_2SO_4$, filtre et concentre. On obtient le composé. Rendement : 95 %.

4.3. 2-[[3-[4-[[5-méthyl-2-(1-méthyléthyl)phénoxy]méthyl]pipéridin-1-yl]propyl]amino]pyrimidine-4-carboxamide.
On fait réagir 4,4 g (0,0144 M) de chlorhydrate de 4-[[5-méthy1-2-(1-méthyléthyl)phénoxy]méthyl]pipéridine-1-propanamine, 2,27 g (0,0144 M) de 2-chloropyrimidine-4-carboxamide et 2,98 g (0,0216 M) de $K_2CO_3$ dans 86 ml de DMF.
On agite le mélange pendant 48 h. On le verse dans de l'eau puis l'extrait 3 fois avec AcOEt. On lave la phase organique 3 fois à l'eau. On sèche sur $Na_2SO_4$, filtre et concentre. Le produit cristallise.
On le reprend dans un peu d'éther et filtre. On obtient 4,5 g (0,0105 M) de base que l'on reprend dans 100 ml d'EtOH et ajoute une solution de 1,22 g (0,0105 M) d'acide fumarique dans 150 ml d'EtOH. F = 183-88°C. Rendement : 36 %.

Exemple 5. 2-[[3-[4-[(5-chloro-2-méthoxyphénoxy)méthyl]pipéridin-1-yl]propyl]amino]pyrimidine-4-carboxamide.

5.1. 4-[(5-chloro-2-méthoxyphénoxy)méthyl]-N-(triphénylméthyl)pipéridine-1-propanamine.
On fait réagir 5,85 g (0,020 M) de chlorhydrate de 4-[(5-chloro-2-méthoxyphénoxy)méthyl]pipéridine, 8,37 g (0,022 M) de 3-bromo-N-(triphénylméthyl)propanamine et 6,91 g (0,050 M) de $K_2CO_3$ dans 50 ml de DMF. On chauffe le mélange réactionnel à 95-100°C. On le refroidit à la température ambiante et le verse sur 150 ml d'eau et l'extrait 3 fois à l'acétate d'éthyle. On réunit les phases organiques, les lave avec de l'eau, les sèche sur $MgSO_4$, filtre et évapore. On obtient le produit après chromatographie.
5.2. Chlorhydrate de 4-[(5-chloro-2-méthoxyphénoxy)méthyl]pipéridine-1-propanamine.
On met en suspension, dans 215 ml de méthanol, 9,5 g (0,017 M) de 4-[(5-chloro-2-méthoxyphénoxy)méthyl]-N-(triphénylméthyl)pipéridine-1-propanamine. On refroidit le mélange réactionnel à 0°C et fait barboter de l'acide chlorhydrique gazeux. On laisse le mélange réactionnel revenir à la température ambiante, on évapore à sec et fait recristalliser le produit obtenu dans un mélange MeOH/AcOEt (50 ml/150 ml). Rendement = 84,9 %. F = 153-157°C.
5.3. 2-[[3-[4-[(5-chloro-2-méthoxyphénoxy)méthyl]pipéridin-1-yl]propyl]amino]pyrimidine-4-carboxamide.
Dans un ballon de 100 ml, on introduit, sous argon, 4,63 g (0,012 M) de chlorhydrate de 4-[(5-chloro-2-méthoxyphénoxy)méthyl]pipéridine-1-propanamine, 1,85 g (0,012 M) de 2-chloropyrimidine-4-carboxamide, 5,8 g (0,042 M) de $K_2CO_3$ et 0,2 g de NaI dans 30 ml de DMF.
On agite le mélange réactionnel à la température ambiante pendant 24 h, le verse sur 100 ml d'eau et l'extrait 3 fois à l'acétate d'éthyle.
On lave les phases organiques avec de l'eau, sèche et évapore. Le produit cristallise.
On ajoute 1,06 g d'acide fumarique à 3,95 g de base dissous dans 50 ml de méthanol. On évapore le méthanol. On fait recristalliser le produit obtenu dans du méthanol. Rendement : 56,4 %. F = 178-180°C.

Exemple 6. 2-[[3-[4-[(4,5-difluoro-2-méthoxyphénoxy)méthyl]pipéridinyl-1-yl]éthyl]amino]pyrimidine-4-carboxamide.

6.1. 4-[(4,5-difluoro-2-méthoxyphénoxy)méthyl]-N-(triphénylméthyl)pipéridine-1-éthanamine.
On fait réagir 5,88 g (0,020M) de chlorhydrate de 4[(4,5-difluoro-2-méthoxyphénoxy)méthyl]pipéridine, 7,7 g (0,021M) de 2-bromo-N-(triphénylméthyl)éthylamine et 6,91 g (0,050M) de $K_2CO_3$ dans 40 ml de DMF. On chauffe le mélange réactionnel à 90°C pendant 8 heures. On le refroidit à la température ambiante, le verse sur 500 ml d'eau et l'extrait 3 fois à l'acétate d'éthyle. On réunit les phases organiques, les lave avec de l'eau, les sèche sur $MgSO_4$, filtre et évapore le solvant sous pression réduite. On obtient le produit sous forme d'huile après chromatographie. Rendement 41,5%.
6.2. Chlorhydrate de 4[(4,5-difluoro-2-méthoxyphénoxy)méthyl]pipéridine-1-éthanamine.
On met en suspension dans 100 ml de méthanol, 4,5 g (0,0083M) de composé 6.1. On refroidit le mélange réactionnel à 0°C et fait passer un courant acide chlorhydrique gazeux. On chauffe le mélange réactionnel à la température du reflux pendant 4 heures puis on le refroidit à la température ambiante. On évapore le solvant sous pression réduite, puis on additionne sur le résidu brut 50 ml d'eau puis 100 ml de NaOH 30% et on extrait au $CH_2Cl_2$. On réunit les phases organiques, les lave avec de l'eau, les sèche sur $MgSO_4$, filtre et évapore sous pression réduite pour obtenir 2,1 g (0,070M) d'huile 6.2. Rendement 84%.
6.3. 2-[[3-[4-[(4,5-difluoro-2-méthoxyphénoxy)méthyl]pipéridinyl-1-yl]éthyl]amino]pyrimidine-4-carboxamide.
Dans un ballon de 100 ml, on introduit 2,1 g (0,007M) de composé 6.2, 1,21g (0,0077M) de 2-chloropyrimidine-4-carboxamide, 1,45 g (0,0105M) de $K_2CO_3$ et 0,1 g de NaI dans 15 ml de DMF.
On agite le mélange réactionnel à la température ambiante pendant 24 heures, le verse sur 100 ml d'eau et l'extrait 3 fois à l'acétate d'éthyle. On lave les phases organiques avec de l'eau, sèche sur $MgSO_4$, filtre et éva-

pore le solvant sous pression réduite. Le résidu brut est chromatographié sur colonne de silice avec comme éluant un mélange $CH_2Cl_2$-MeOH (98-2 à 92-8) pour donner 1,38 g (0,0033M) de base.
On ajoute 0,38 g d'acide fumarique à 1,38 g de base dissous dans 10 ml d'éthanol puis on évapore le solvant sous pression réduite. On fait recristalliser le produit obtenu dans un mélange méthanol-éthanol pour obtenir 1,35 g (0,0025M) Rendement : 36%. F= 211-215°C (décomposition)

Exemple 7. [N-[2-[4-(4-fluorophénoxy)pipéridin-1-yl]éthyl-] N-méthyl-amino]pyrimidine-4-carboxamide.

7.1. 4-(4-fluoro-phénoxy)-N-(triphénylméthyl)-N-méthylpipéridine-1-éthylamine.

Dans un tricol de 500 ml, on introduit 5,25 g (0,0227M) de chlorhydrate de 4-(4-fluorophénoxy)pipéridine, 9,5 g (0,025M) de 2-bromo-N-(triphénylméthyl)-N-méthyléthylamine et 7,85 g (0,0568M) de $K_2CO_3$ dans 50 ml de DMF puis on chauffe le mélange réactionnel à 90-100°C pendant 7 heures. On le refroidit à la température ambiante, on le verse sur 200 ml d'eau et on extrait 3 fois à l'acétate d'éthyle. On lave les phases organiques avec de l'eau, sèche sur $MgSO_4$, filtre puis évapore le solvant sous pression réduite. On obtient après chromatographie sur colonne de silice avec comme éluant un mélange $CH_2Cl_2$-MeOH (98-2) 8 g (0,016M) de produit sous forme d'huile. Rendement 71,4%.

7.2. Dichlorhydrate de N-méthyl-4-(4-fluorophénoxy)pipéridine-1-éthylamine.

On met en suspension dans 160 ml de méthanol, 7,7 g (0,0156M) de composé 7.1. On refroidit le mélange réactionnel à 0°C et fait passer un courant acide chlorhydrique gazeux. On chauffe le mélange réactionnel à la température du reflux pendant 7 heures puis on le refroidit à la température ambiante. Le solvant est évaporé sous pression réduite puis on fait cristalliser le produit par addition d'acétate d'éthyle. On obtient 4,65 g (0,0143M) de dichlorhydrate 7.2. Rendement 92% F = 212-214,5°C.

7.3. [N-[2-[4-(4-fluorophénoxy)pipéridin-1-yl]éthyl]-N-méthyl-amino]pyrimidine-4-carboxamide.

Dans un ballon de 500 ml, on introduit, sous argon, 4,65 g (0,0143M) de dichlorhydrate 7.2, 2,33 g (0,0148M) de 2-chloropyrimidine-4-carboxamide, 6,9 g (0,050M) de $K_2CO_3$ et 0,2 g de NaI dans 75ml de DMF. On agite le mélange réactionnel à la température ambiante pendant 24 heures, le verse sur 150 ml d'eau et l'extrait trois fois à l'acétate d'éthyle. On lave les phases organiques avec de l'eau, sèche sur $MgSO_4$, filtre puis évapore le solvant sous pression réduite. On fait cristalliser le produit par addition d'éther pour obtenir 4,1 g (0,011M) de base. On ajoute 1,28 g d'acide fumarique à 4,1 g de base dissous dans 200 ml d'éthanol puis on évapore sous pression réduite. On fait recristalliser le produit dans l'éthanol pour obtenir 3,3 g (0,0068M). Rendement = 47%. F = 196-198,5°C.

Le tableau suivant illustre les structures et les propriétés physiques de quelques composés selon l'invention.

TABLEAU

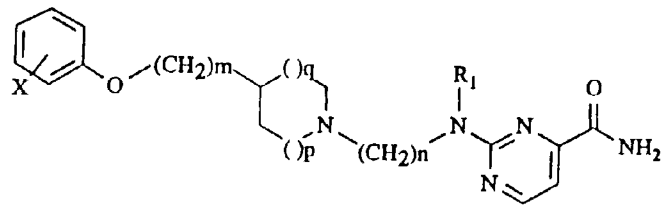

| Composé | X | m | p | q | n | $R_1$ | $[\alpha]_D^{25}$ (°) | sel | F(°C) |
|---|---|---|---|---|---|---|---|---|---|
| 1 | H | 0 | 0 | 2 | 3 | H | rac | fum | 135-138 |
| 2 | 2-$OCH_3$ | 0 | 0 | 2 | 3 | H | rac | fum | 148-151 |
| 3 | H | 0 | 1 | 1 | 3 | H | rac | fum | 185-187 |
| 4 | 4-F | 0 | 1 | 1 | 3 | H | rac | fum | 171-174 |
| 5 | 2-$OCH_3$ | 0 | 1 | 1 | 3 | H | rac | fum | 157-160 |
| 6 | 2-i$C_3H_7$, 5-$CH_3$ | 0 | 1 | 1 | 3 | H | rac | fum | 184-187 |
| 7 | 2-$OCH_3$, 5-Cl | 0 | 1 | 1 | 3 | H | rac | fum | 199-201 |
| 8 | 2-$OCH_3$, 5-F | 0 | 1 | 1 | 3 | H | rac | fum | 191,5-192,5 |
| 9 | 4-F | 0 | 1 | 1 | 2 | H | rac | fum | 245-247,5 |
| 10 | 4-F | 0 | 1 | 1 | 2 | $CH_3$ | rac | fum | 196-198,5 |

| Composé | X | m | p | q | n | $R_1$ | $[\alpha]_D^{25}$ (°) | sel | F(°C) |
|---|---|---|---|---|---|---|---|---|---|
| 11 | H | 1 | 1 | 1 | 3 | H | rac | ½fum | 190-193 |
| 12 | 2-$OCH_3$ | 1 | 1 | 1 | 3 | H | rac | chl | 198-201 |
| 13 | 2-i$C_3H_7$,5-$CH_3$ | 1 | 1 | 1 | 3 | H | rac | fum | 183-188 |
| 14 | 2-$OCH_3$,5-Cl | 1 | 1 | 1 | 3 | H | rac | fum* | 178-180 |
| 15 | 2-$OCH_3$,5-F | 1 | 1 | 1 | 3 | H | rac | fum | 167-169 |
| 16 | 2-$OCH_3$,4,5-$F_2$ | 1 | 1 | 1 | 3 | H | rac | ½fum | 185-188 |
| 17 | 2-i$C_3H_7$ | 1 | 1 | 1 | 3 | H | rac | fum | 194-196(dec) |
| 18 | 2-i$C_3H_7$,4-F,5-$CH_3$ | 1 | 1 | 1 | 3 | H | rac | fum | 195-198 |
| 19 | 2-$OCH_3$,5-Cl | 1 | 1 | 1 | 2 | H | rac | fum | 228-232(dec) |
| 20 | 2-$OCH_3$,4,5-$F_2$ | 1 | 1 | 1 | 2 | H | rac | fum | 211-215(dec) |
| 21 | 2-$OCH_3$,4,5-$F_2$ | 1 | 1 | 1 | 2 | $CH_3$ | rac | fum | 188-192(dec) |
| 22 | H | 1 | 0 | 2 | 3 | H | rac | fum | 144-146 |
| 23 | H | 1 | 0 | 2 | 3 | H | +4,48(c=1,MeOH) | fum | 150,5-152,5 |
| 24 | H | 1 | 0 | 2 | 3 | H | -6(c=1,MeOH) | fum | 151-153 |
| 25 | 4-F | 1 | 0 | 2 | 3 | H | rac | fum | 150-153 |
| 26 | 2-$OCH_3$ | 1 | 0 | 2 | 3 | H | rac | fum | 137-142 |

| Composé | X | m | p | q | n | $R_1$ | $[\alpha]_D^{25}$ (°) | sel | F(°C) |
|---|---|---|---|---|---|---|---|---|---|
| 27 | 2-i$C_3H_7$,5-$CH_3$ | 1 | 0 | 2 | 3 | H | rac | fum | 141-148 |
| 28 | 2-$OCH_3$,5-Cl | 1 | 0 | 2 | 3 | H | rac | fum | 139-141 |
| 29 | 2-$OCH_3$,4,5-$(F)_2$ | 1 | 0 | 2 | 3 | H | rac | $\frac{1}{2}$fum | 183-186 |
| 30 | 2-i$C_3H_7$,5-$CH_3$ | 1 | 0 | 2 | 3 | $CH_3$ | rac | chl | 138-143 |
| 31 | 4-F | 1 | 0 | 2 | 2 | H | rac | fum | 210-213 |
| 32 | 4-F | 1 | 0 | 2 | 2 | $CH_3$ | rac | fum | 171-173,5 |

Note : fum désigne un fumarate
1/2 fum désigne un hémifumarate
chl désigne un chlorhydrate
fum* désigne un fumarate hydraté
dec signifie une décomposition

Les composés de l'invention ont été soumis à une série d'essais pharmacologiques qui ont mis en évidence leur intérêt comme substances à activités thérapeutiques.

Les composés de l'invention ont fait l'objet d'études quant à leur activité antagoniste des récepteurs adrénergiques du type $\alpha_1$ au niveau du bas appareil urinaire.

Leur activité *in vitro* a été étudiée sur l'urètre isolé de lapin.

On prépare des anneaux d'urètre de lapin adulte selon la méthode de Ueda et al., Eur. J. Pharmacol., (1984), 103, 249-254, puis, après sensibilisation à la noradrénaline, on détermine la courbe concentration-réponse à la phényléphrine, en absence et en présence de composé à étudier.

On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.

Les $pA_2$ des composés sont de l'ordre de 5,5 à 9.

L'activité *in vivo* des composés de l'invention a été étudiée quant à leur effet sur l'hypertonie urétrale engendrée par la stimulation du nerf hypogastrique chez le chat anesthésié. On anesthésie des chats mâles adultes au pentobarbital sodique, et on les prépare selon la méthode de Theobald, J. Auton. Pharmac., (1983), 3, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 μg/kg.

On évalue la puissance de l'antagonisme $\alpha_1$-adrénergique de chaque composé par calcul de la $DI_{50}$, dose qui inhibe de 50% l'hypertonie urétrale.

Les $DI_{50}$ des composés de l'invention sont de l'ordre de 0,01 à 3 mg/kg.

Les résultats des essais montrent que certains composés de l'invention possèdent *in vitro*, une activité antagoniste des récepteurs $\alpha_1$ adrénergique des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 100 mg de substance active.

## ANNEXE 1

### Schéma 1

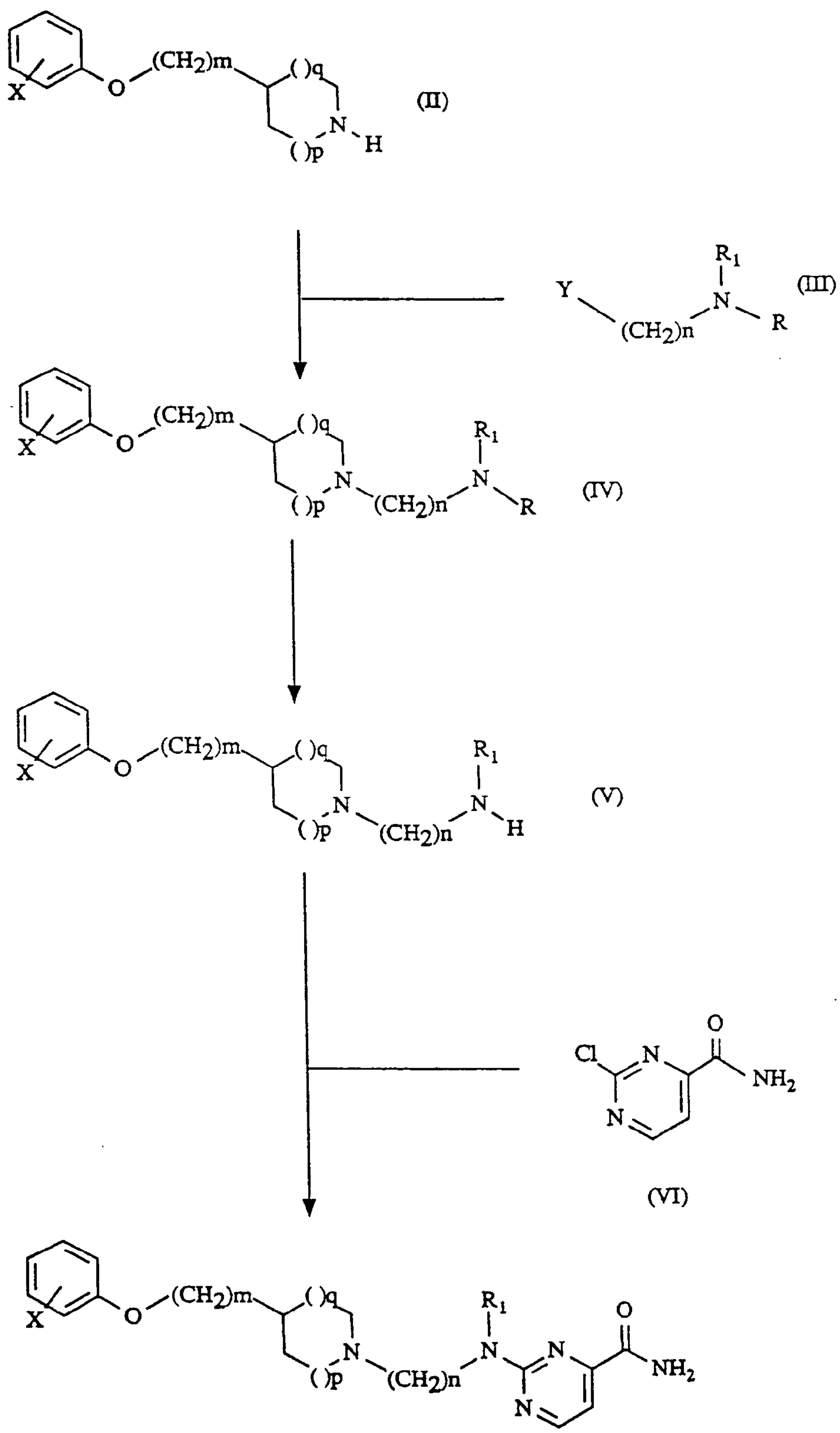

(II)

(III)

(IV)

(V)

(VI)

(I)

## Revendications

1. Dérivés de 2-aminopyrimidine-4-carboxamide, sous la forme de racémates ou d'énantiomères, répondant à la formule (I)

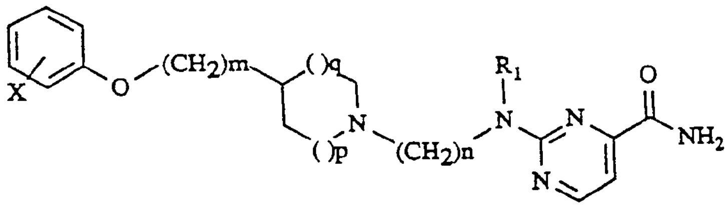

(I)

dans laquelle
m représente le nombre 0 ou 1,
p représente le nombre 0 ou 1,
q représente le nombre 1 ou 2,
n représente le nombre 2 ou 3,
$R_1$ représente un atome d'hydrogène ou un groupe méthyle, et
X représente un ou plusieurs atomes ou groupes choisis parmi les suivants : hydrogène, fluor, chlore, méthoxy, méthyle, isopropyle,
ainsi que leurs sels d'addition aux acides pharmaceutiquement acceptables.

2. Procédé de préparation des dérivés selon la revendication 1, procédé caractérisé en ce que l'on fait réagir une amine de formule (II)

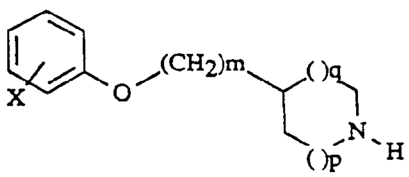
(II)

éventuellement sous forme de chlorhydrate, avec un réactif halogéné de formule (III)

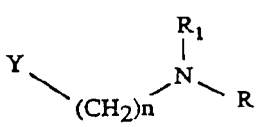
(III)

dans un solvant aprotique, en présence d'une base organique ou minérale, à une température de 40 à 100°C et on déprotège l'alkylamine terminale de la diamine de formule (IV)

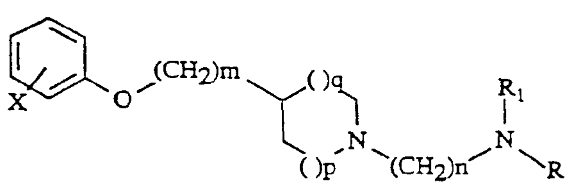
(IV)

soit par traitement à l'acide chlorhydrique gazeux, dans un alcool aliphatique, à une température de 0 à 60°C soit par traitement par exemple avec l'hydrazine, puis on fait réagir l'amine de formule (V)

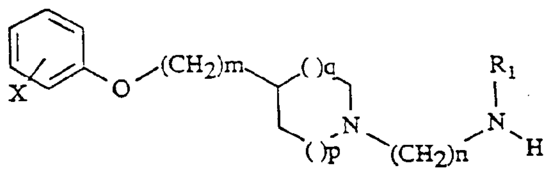

(V)

avec un 2-chloropyrimidine-4-carboxamide de formule (VI)

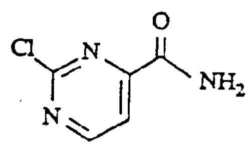

dans un solvant aprotique, en présence d'une base, à une température de 20 à 40°C pour arriver au dérivé de 2-aminopyrimidine-4-carboxamide de formule (I),
les indices m, p, q, n, et les radicaux $R_1$, et X ayant les significations données ci-dessus,
Y représente un atome d'halogène,
R représente un groupe protecteur de l'amine par exemple un groupe triphénylméthyle,
ou R forme avec $R_1$ un groupe protecteur tel que le groupe phtalimido.

3. Médicament, caractérisé en ce qu'il contient un composé selon la revendication 1.

4. Composition pharmaceutique caractérisée en ce qu'elle contient un composé selon la revendication 1, associé à un excipient convenable.

**Revendications pour les Etats contractants suivants: ES - GR**

1. Procédé de préparation des dérivés de 2-aminopyrimidine-4-carboxamide, sous la forme de racémates ou d'énantiomères, répondant à la formule (I)

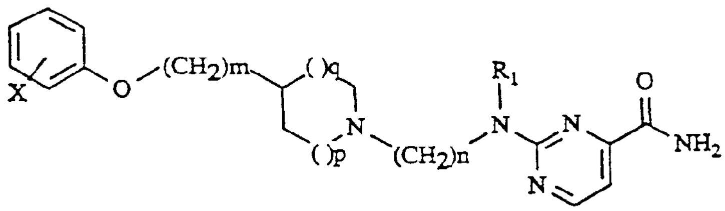

(I)

dans laquelle
m représente le nombre 0 ou 1,
p représente le nombre 0 ou 1,
q représente le nombre 1 ou 2,
n représente le nombre 2 ou 3,
$R_1$ représente un atome d'hydrogène ou un groupe méthyle, et
X représente un ou plusieurs atomes ou groupes choisis parmi les suivants : hydrogène, fluor, chlore, méthoxy, méthyle, isopropyle,
ainsi que de leurs sels d'addition aux acides pharmaceutiquement acceptables,
procédé caractérisé en ce que l'on fait réagir une amine de formule (II)

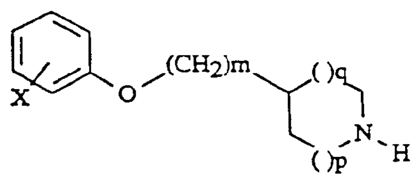

(II)

éventuellement sous forme de chlorhydrate, avec un réactif halogéné de formule (III)

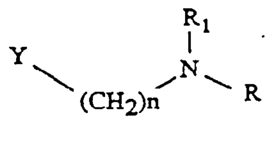 (III)

dans un solvant aprotique, en présence d'une base organique ou minérale, à une température de 40 à 100°C et on déprotège l'alkylamine terminale de la diamine de formule (IV)

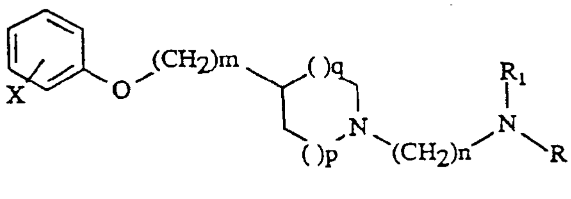 (IV)

soit par traitement à l'acide chlorhydrique gazeux, dans un alcool aliphatique, à une température de 0 à 60°C soit par traitement par exemple avec l'hydrazine, puis on fait réagir l'amine de formule (V)

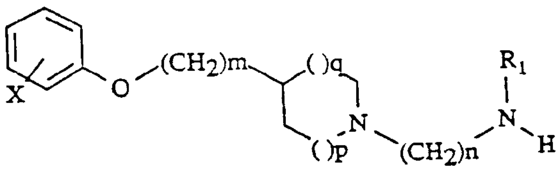 (V)

avec un 2-chloropyrimidine-4-carboxamide de formule (VI)

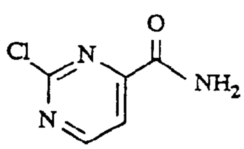

dans un solvant aprotique, en présence d'une base, à une température de 20 à 40°C pour arriver au dérivé de 2-aminopyrimidine-4-carboxamide de formule (I),
les indices m, p, q, n, et les radicaux $R_1$, et X ayant les significations données ci-dessus,
Y représente un atome d'halogène,
R représente un groupe protecteur de l'amine par exemple un groupe triphénylméthyle,
ou R forme avec $R_1$ un groupe protecteur tel que le groupe phtalimido.

Office européen des brevets

# RAPPORT DE RECHERCHE EUROPEENNE

Numero de la demande

EP 91 40 2604

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 301 936 (SYNTHELABO)<br>* page 1, ligne 5-12; revendications 1,3,4 *<br>--- | 1,3,4 | C07D401/12<br>A61K31/505 |
| A | US-A-3 290 317 (P.M. CARABATEAS)<br>* colonne 2, lignes 28-46; colonne 6, ligne 53; colonne 13, exemple 26 *<br>--- | 1 | |
| A | DE-A-2 143 730 (BYK GULDEN LOMBERG)<br>* pages 1-2; page 6 *<br>--- | 1,3,4 | |
| A | EP-A-0 389 352 (ADIR)<br>* page 3 - page 4; exemples 7,20,21 *<br>----- | 1,3,4 | |
| | | | **DOMAINES TECHNIQUES RECHERCHES (Int. Cl.5)** |
| | | | C07D |

Le présent rapport a été établi pour toutes les revendications

| Lieu de la recherche | Date d'achèvement de la recherche | Examinateur |
|---|---|---|
| LA HAYE | 07 JANVIER 1992 | RUSSELL F. ENGLISH |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)